# EUROPEAN PATENT APPLICATION

(11) **EP 1 908 766 A1**
(43) Date of publication of application: **09.04.2008**
(21) Application number: 06768355.7
(22) Date of filing: 21.07.2006
(51) Int. Cl.: C07D 513/04

(54) **PROCESS FOR PRODUCTION OF IMIDAZOTHIAZOLE DERIVATIVE**

(30) Priority: 22.07.2005 JP 2005213082
(71) Applicant: Meiji Seika Kaisha, Ltd., Tokyo 104-8002 (JP)
(72) Inventor: KUBOTA, Dai, Kouhoku-ku, Yokohama-shi, Kanagawa 222-8567 (JP); YAMAMOTO, Yasuo, Kouhoku-ku, Yokohama-shi, Kanagawa 222-8567 (JP); SASAKI, Toshiro, Kouhoku-ku, Yokohama-shi, Kanagawa 222-8567 (JP); ANDO, Takashi, Kouhoku-ku, Yokohama-shi, Kanagawa 228-8567 (JP); SHITARA, Eiki, Kouhoku-ku, Yokohama-shi, Kanagawa 228-8567 (JP); SAKATA, Katsuya, 2750026 (JP); SUZUKI, Kenji, 2750026 (JP)
(74) Representative: Warcoin, Jacques
(86) International application number: PCT/JP2006/314477
(87) International publication number: WO 2007/011021

(57) **Abstract**

Disclosed is a process for producing an imidazothiazole derivative of formula (I) useful as an intermediate for the production of carbapenem derivatives having potent antimicrobial activity and a broad antimicrobial spectrum, that is, a novel method for nicotinoylating an imidazo[5,1-b]thiazole ring at its 7-position. The process comprises reacting a compound of formula (II) with a compound of formula (III).

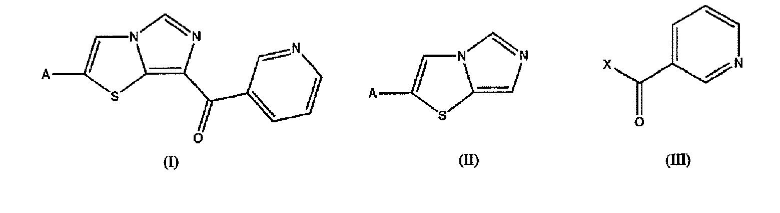

## Description

### FIELD OF THE INVENTION

The present invention relates to a process for producing imidazothiazole derivatives useful as an intermediate for the production of carbapenem derivatives.

### BACKGROUND ART

Since Carbapenem derivatives have potent antimicrobial activity and a broad antimicrobial spectrum, they have been energetically studied as a highly useful β-lactam agent.

In WO 2002/42312, the present inventors report finding that carbapenem derivatives having a 7-(1-carbamoylmethylpyridinium-3-yl)carbonylimidazo[5,1-b]thiazole group at the 2-position on the carbapenem ring (compounds of formula (A) in the following scheme) have high antimicrobial activity against Gram-positive bacteria and Gram-negative bacteria including MRSA (methicillin-resistant Staphylococcus aureus), PRSP (penicillin resistant Streptococcus pneumoniae), Haemophilus influenzae, and β-lactamase producing bacteria, and, at the same time, have high stability against DHP-1 (kidney dehydropeptidase-1).

Further, in WO 2004/055027, the present inventors report processes represented by scheme A and scheme B as a process for producing a compound of formula (A). In scheme A and scheme B, respectively a compound of formula (B) and a compound of formula (C) are reported as important intermediates.

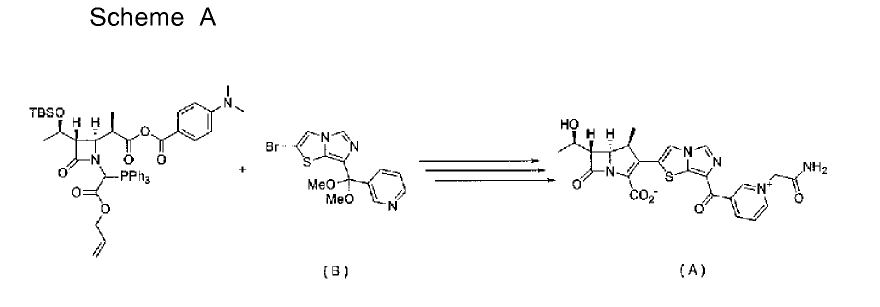

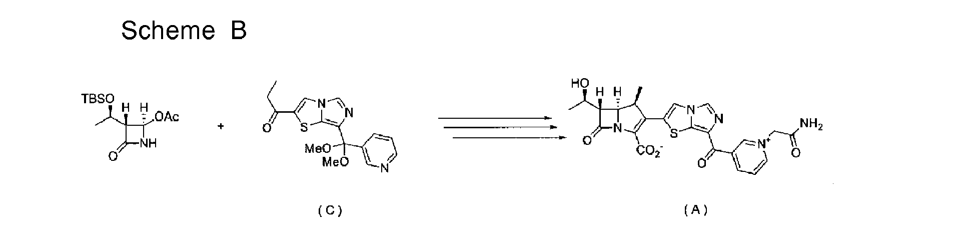

Further, WO 2004/055027 discloses the following scheme C as a process for producing compounds of formulae (B) and (C).

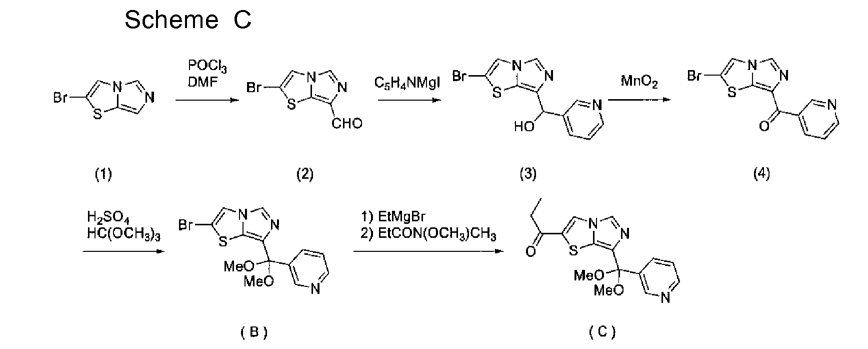

In the process represented by scheme C, a compound of formula (B) is produced from 2-bromoimidazo[5,1-b]thiazole (a compound of formula (1)) through four steps, and a compound of formula (C) is produced from the compound of formula (B) through one step.

In the process represented by scheme C, however, there is room for improvement in production cost and yield due to a long process necessary for achieving the nicotinoylation of the compound of formula (1) at its 7-position (that is, until a compound of formula (4) is obtained). That is, a process for producing a compound of formula (4), i.e., a method for nicotinoylating a compound of formula (1) at its 7-position, which requires a smaller number of steps, can realize a reduction in production cost, and an improvement in operationality, and has higher safety, has been desired.

On the other hand, Journal of Organic Chemistry, 1977, 4248 discloses the nicotinoylation of a pyrrole ring using N,N-diethylnicotinamide and phosphorus oxychloride. It is however difficult to say that the same reaction will proceed in an imidazo[5,1-b]thiazole ring having a different ring construction.

Further, Journal of Medicinal Chemistry, 1986, 860 discloses the nicotinoylation of imidazolone using nicotinoyl chloride and aluminum chloride. However, it is also difficult to say that the same reaction will proceed in an imidazo[5,1-b]thiazole ring having a different ring construction.

### DISCLOSURE OF INVENTION

The present inventors have now found a novel method for the nicotinoylation of an imidazo[5,1-b]thiazole ring at its 7-position which is a key reaction in the synthesis of a carbapenem derivative. This method can realize the nicotinoylation of an imidazo[5,1-b]thiazole ring at its 7-position basically through one step and thus is efficient and low in cost. The present invention has been made based on such finding.

Thus, according to the present invention, there is provided a process for producing a compound of formula (I) 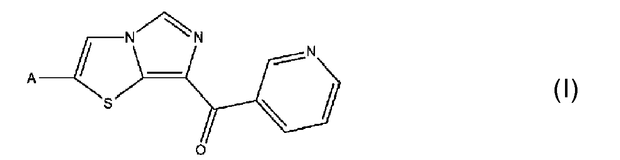 wherein A represents a hydrogen atom, a halogen atom, or -COR¹ wherein R¹ represents C₁₋₁₂ alkyl or C₁₋₁₂ alkyloxy, the process comprising reacting a compound of formula (II): 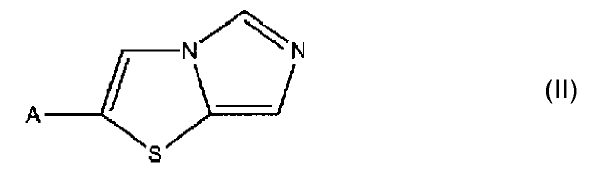 wherein A is as defined above, with a compound of formula (III): 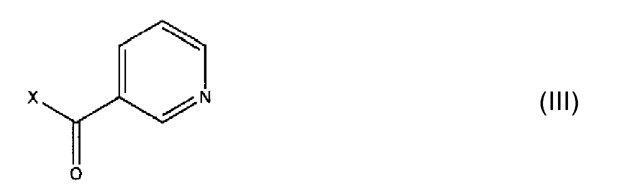 wherein X represents -NR²R³ wherein R² and R³, which may be the same or different, represent C₁₋₁₂ alkyl, or R² and R³ together represent - (CH₂)n- wherein n is an integer of 2 to 8; or a halogen atom.

### DETAILED DESCRIPTION OF THE INVENTION

In the production process according to the present invention, a compound of formula (I) is produced by reacting a compound of formula (II) with a compound of formula (III). According to the present invention, the compound of formula (I) can be produced through one step, and the production time can be shortened, for example, to not more than one-third of the time required in the prior art. Further, the yield and operationality in the process according to the present invention is more advantageous than those in the conventional method.

The production process according to the present invention may be divided into two embodiments.

### Reaction of compound of formula (II) with compound of formula (III) wherein X represents -NR²R³ (step (a))

According to an embodiment of the present invention, a compound of formula (II) is reacted with a compound of formula (III) wherein X represents -NR²R³ to give a compound of formula (I). This reaction will be hereinafter referred to as step (a).

The reaction may be carried out in an inert solvent to the reaction or in the absence of a solvent and in the presence of an inorganic acid. The reaction is preferably carried out in the presence of a halogenating reagent.

The solvent used in the reaction is not particularly limited so far as the solvent is inert to the reaction. Specific examples thereof include methylene chloride, chloroform, 1,2-dichloroethane, 1,4-dioxane, acetonitrile, propionitrile, butyronitrile, and nitrobenzene. These solvents may be used either solely or as a mixed solvent. The solvent is preferably 1,2-dichloroethane, 1,4-dioxane, butyronitrile, or nitrobenzene.

Halogenating reagents include phosphorus oxychloride, phosphorus oxybromide, phosphorus pentachloride, oxalyl chloride, and thionyl chloride. The halogenating reagent is preferably phosphorus oxychloride. The amount of the halogenating reagent used is preferably 1 to 100 molar equivalents.

While the reaction temperature may vary depending upon the solvent and the like, it is generally 0°C to 200°C. While the reaction time may also vary depending upon the solvent and reaction temperature used, it is generally 10 min to 24 hr.

The compound of formula (I) is produced by carrying out conventional general treatment. Further, the compound of formula (I) may be purified, for example, by precipitation or column chromatography on silica gel. Alternatively, the compound of formula (I) may be used in a next step without the purification.

### Reaction of compound of formula (II) with compound of formula (III) wherein X represents halogen atom (step (b))

According to another embodiment of the present invention, a compound of formula (II) is reacted with a compound of formula (III) wherein X represents halogen atom to give a compound of formula (I). This reaction will be hereinafter referred to as step (b).

The reaction may be carried out in a solvent inert to the reaction or in the absence of a solvent and in the presence of a Lewis acid.

The solvent used in the reaction is not particularly limited so far as the solvent is inert to the reaction. Specific examples thereof include methylene chloride, chloroform, 1,2-dichloroethane, acetonitrile, propionitrile, butyronitrile, nitromethane, nitrobenzene, and o-dichlorobenzene. These solvents may be used either solely or as a mixed solvent. The solvent is preferably 1,2-dichloroethane or butyronitrile.

Lewis acids include aluminum chloride, titanium tetrachloride, tin chloride, zinc chloride, iron chloride, boron trifluoride, and boron tribromide. Preferred are aluminum chloride, titanium tetrachloride, and tin chloride. More preferred is titanium tetrachloride. The amount of the Lewis acid is preferably 1 to 100 molar equivalents.

While the reaction temperature may vary depending upon the solvent and the like, it is generally 0°C to 200°C. While the reaction time may also vary depending upon the solvent and reaction temperature used, it is generally 10 min to 120 hr.

The compound of formula (I) can be produced by carrying out conventional general treatment. Further, the compound of formula (I) may be produced as a hydrohalide salt by adding a lower alcohol to the reaction solution in the post treatment. Lower alcohols include methanol, ethanol, n-propyl alcohol, isopropyl alcohol, and butanol. Preferred are methanol and ethanol. The hydrohalide salt corresponds to a Lewis acid. Preferred are hydrochloride and hydrobromide. Further, purification may be carried out by a method such as precipitation or column chromatography on silica gel. Alternatively, the compound may be used in a next step without purification.

### Compounds of formulae (I), (II), and (III)

The term "alkyl" as used herein as a group or a part of a group in formulae (I), (II), and (III) in the specification means alkyl which is of a straight chain, branched chain, or cyclic type or a combination thereof unless otherwise specified. For example, "C₁₋₁₂" in "C₁₋₁₂ alkyl" means that the number of carbon atoms in the alkyl group is 1 to 12.

Specific examples of alkyl include methyl, ethyl, n-propyl, i-propyl, n-butyl, i-butyl, s-butyl, t-butyl, pentyl, hexyl, octyl, nonyl, decyl, dodecyl, cyclopropyl, cyclobutyl, cyclopentyl, cyclohexyl, cycloheptyl, and cyclooctyl. In the present invention, the alkyl group is preferably C₁₋₆ alkyl, more preferably C₁₋₄ alkyl. Examples thereof include methyl, ethyl, n-propyl, i-propyl, n-butyl, i-butyl, s-butyl, t-butyl, cyclopropyl, and cyclobutyl.

Likewise, in formulae (I), (II), and (III), the term "alkyl" in the term "alkyloxy" as used herein as a group or a part of a group means alkyl which is of a straight chain, branched chain, or cyclic type or a combination thereof unless otherwise specified. Further, "C₁₋₁₂" in "C₁₋₁₂ alkyloxy" means that the number of carbon atoms in the alkyl group is 1 to 12.

Specific examples of alkyloxy include methyloxy, ethyloxy, n-propyloxy, i-propyloxy, n-butyloxy, i-butyloxy, s-butyloxy, t-butyloxy, pentyloxy, hexyloxy, octyloxy, nonyloxy, decyloxy, dodecyloxy, cyclopropyloxy, cyclobutyloxy, cyclopentyloxy, cyclohexyloxy, cycloheptyloxy, and cyclooctyloxy. In the present invention, the alkyloxy group is preferably C₁₋₆ alkyl, more preferably C₁₋₄ alkyl, and examples thereof include methyloxy, ethyloxy, n-propyloxy, i-propyloxy, n-butyloxy, i-butyloxy, s-butyloxy, and t-butyloxy.

In the present invention, the alkyl and alkyloxy groups may be optionally substituted. Specifically, one or more hydrogen atoms on the alkyl or alkyloxy group may be substituted by one or more substituents which may be the same or different. Specific examples of these substituents include, for example, halogen atoms and alkyloxy, amino, and hydroxyl groups.

In formulae (I) and (II) in the present specification, A represents a hydrogen or halogen atom, or -COR¹, more preferably a hydrogen atom, a chlorine atom, a bromine atom, or -COR¹, still preferably a bromine atom or -COR¹. Here R¹ represents C₁₋₆ alkyl or C₁₋₆ alkyloxy, more preferably C₁₋₄ alkyl or C₁₋₄ alkyloxy, still more preferably methyl, ethyl, methyloxy, or ethyloxy.

Further, in formula (III), X represents -NR²R³ or a halogen atom. Preferably, R² and R³, which may be the same or different, represent C₁₋₆ alkyl, or R² and R³ together represent -(CH₂)n- wherein n is an integer of 2 to 6. More preferably, R² and R³, which may be the same or different, represent C₁₋₄ alkyl, or R² and R³ together represent - (CH₂)n- wherein n is an integer of 4 to 6. Still more preferably, R² and R³ which may be the same or different, represent methyl or ethyl. X preferably represents a chlorine atom or a bromine atom, more preferably a chlorine atom.

The term "halogen atom" as used herein means a fluorine, chlorine, bromine, or iodine atom. The term "amino" as used herein represents unsubstituted amino, dialkylamino, or cyclic alkylamino.

Specific examples of preferred compounds of formula (I) include 7-(pyridin-3-yl)carbonylimidazo[5,1-b]thiazole, 2-bromo-7-(pyridin-3-yl)carbonylimidazo[5,1-b]thiazole, 2-ethoxycarbonyl-7-(pyridin-3-yl)carbonylimidazo[5,1-b]thiazole, and 2-propionyl-7-(pyridin-3-yl)carbonylimidazo[5,1-b]thiazole.

Further, specific examples of preferred compounds of formula (II) include imidazo[5,1-b]thiazole, 2-bromo-imidazo[5,1-b]thiazole, 2-ethoxycarbonyl-imidazo[5,1-b]thiazole, and 2-propionyl-imidazo[5,1-b]thiazole.

Specific examples of preferred compounds of formula (III) include N,N-dimethylnicotinamide, N,N-diethylnicotinamide, N,N-diisopropylnicotinamide, and nicotinoyl chloride.

### Production of compounds of formula (II)

In the process according to the present invention, not only compounds of formula (II) wherein A represents a halogen atom but also compounds of formula (II) wherein A represents -COR¹ may be utilized. The latter compounds are available or can be synthesized more inexpensively than the former compounds. Accordingly, the process for producing a compound of formula (I) from a compound of formula (II) wherein A represents -COR¹, according to the present invention, is advantageous from the viewpoint of production cost.

For example, as described in scheme D, a compound of formula (I) wherein A represents -COR¹ (preferably, R¹ = ethyl) (a compound of formula (e) in the scheme) can be synthesized through a compound of formula (II) wherein A represents -COR¹ (preferably, R¹ = ethyl) (a compound of formula (d) in the scheme).

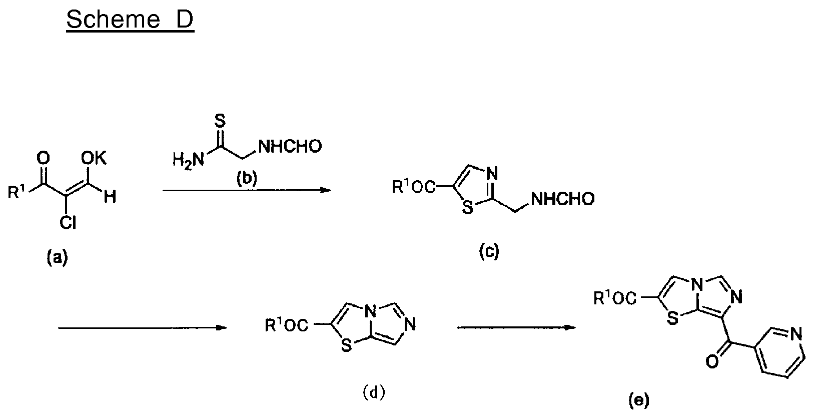

### Synthesis of carbapenem derivative

Carbapenem derivatives may be synthesized from the compound of formula (I), for example, according to the following scheme.

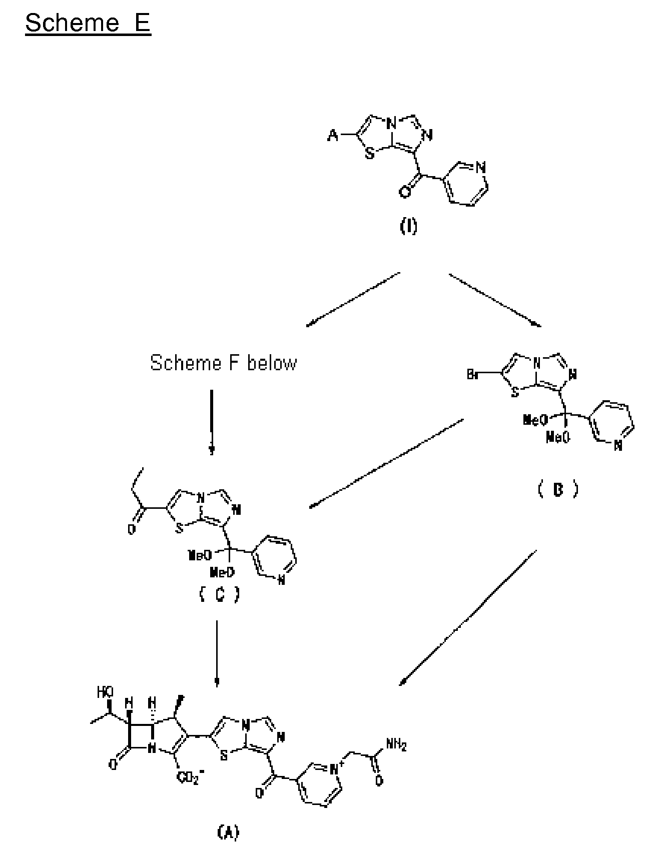

A compound of formula (A), that is, a carbapenem derivative, can be produced according to a method described in WO 2004/055027 by first leading the compound of formula (I) to a compound of formula (B) and then leading the compound of formula (B) to the compound of formula (A) through or without through a compound of formula (C).

A compound of formula (e) in scheme D (that is, a compound of formula (I) wherein A represents -COR¹ (preferably, R¹ = ethyl)) can be converted to a compound of formula (C) by a method as shown in the following scheme.

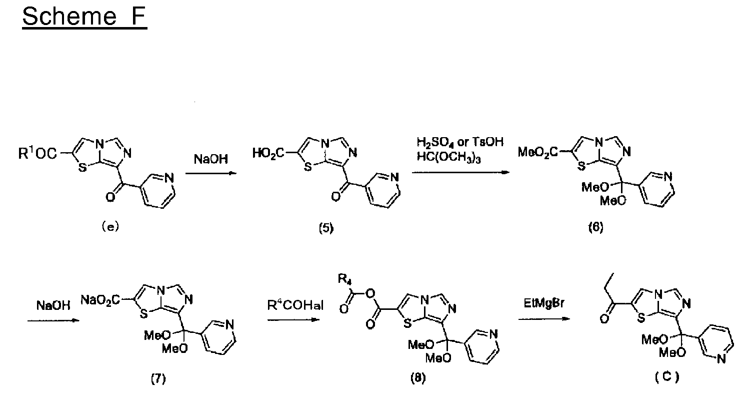

In the above scheme, R⁴ represents C₁₋₁₂ alkyl or aryl, and X represents a halogen atom. The aryl group is preferably a six- to fourteen-membered aromatic ring (mono- to tricyclic). Specific examples thereof include phenyl, 1-naphthyl, 2-naphthyl, biphenyl, and 2-anthryl naphthyl. Preferred is phenyl. The aryl group is optionally substituted. One or more hydrogen atoms on aryl are optionally substituted by one or more substituents which may be the same or different. Specific examples of such substituents include halogen atoms, alkyl, alkyloxy, amino, and hydroxyl.

In the above scheme, a compound of formula (6) is produced by treating a compound of formula (e) (that is, a compound of formula (I) wherein A represents -COR¹ (preferably, R¹ = ethyl)), if necessary, with a hydrolysis reagent such as sodium hydroxide and then allowing a reaction to proceed in the presence of an acid catalyst in a methanol solvent. Acid catalysts usable in this step include sulfuric acid, hydrochloric acid, p-toluenesulfonic acid, and methanesulfonic acid. Preferred are sulfuric acid and p-toluenesulfonic acid. The amount of the acid catalyst used is preferably 1 to 100 molar equivalents.

Whiel the reaction temperature may vary depending upon the solvent and the like, it is generally 0°C to 100°C. While the reaction time may also vary depending, for example, upon the solvent and reaction temperature used, it is generally 10 min to 48 hr.

Preferably, water is removed from the reaction system. To this end, dehydrating agents such as molecular sieves, silica gel, anhydrous magnesium sulfate, anhydrous sodium sulfate, and methyl orthoformate are added. Alternatively, water may be removed by reflux with a dehydrator such as Dienstag. Preferred dehydrating agents include molecular sieves and methyl orthoformate. More preferred is methyl orthoformate.

A mixed acid anhydride of formula (8) may be produced by reacting a compound of formula (6) with a compound of R⁴COHal, if necessary, after the treatment of the compound of formula (6) with a hydrolysis reagent such as sodium hydroxide. A compound of formula (C) is produced by reacting this product with ethylmagnesium bromide. Here R⁴ represents C₁₋₁₂ alkyl or aryl, preferably aryl, more preferably phenyl in which one or more hydrogen atoms on the group are optionally substituted, still more preferably 4-dimethylaminophenyl or 4-diethylaminophenyl. Hal represents a halogen atom.

Specific examples of compounds represented by R⁴COHal include acetyl chloride, pivaloyl chloride, benzoyl chloride, 4-dimethylaminobenzoyl chloride, and 4-diethylaminobenzoyl chloride. Preferred are pivaloyl chloride, benzoyl chloride, 4-dimethylaminobenzoyl chloride, and 4-diethylaminobenzoyl chloride. More preferred are 4-dimethylaminobenzoyl chloride and 4-diethylaminobenzoyl chloride.

The compounds of formulae (B) and (C) can be produced by carrying out conventional post treatment. Further, the compounds may be purified, for example, by precipitation or column chromatography on silica gel. Alternatively, the compounds may be used in a next step without the purification.

### EXAMPLES

### Example 1

### 7-(Pyridin-3-yl)carbonylimidazo[5,1-b]thiazole (step (a))

N,N-Dimethylnicotinamide (600 mg, 4.00 mmol) was dissolved in 1,2-dichloroethane (1.0 ml) under an argon atmosphere, and phosphorus oxychloride (1.25 g, 8.15 mmol) was added dropwise to the solution at room temperature. A 1,2-dichloroethane solution (1.0 ml) of imidazo[5,1-b]thiazole (250 mg, 2.00 mmol) was added thereto, and the mixture was refluxed for 16 hr. A 1 N aqueous sodium hydroxide solution was added to stop the reaction, and the reaction mixture was extracted with dichloroethane. The organic layer was dried over anhydrous magnesium sulfate and was concentrated under the reduced pressure. The residue was purified by column chromatography on silica gel (development system: ethyl acetate/methanol = 10/1) to give 7-(pyridin-3-yl)carbonylimidazo[5,1-b]thiazole (160 mg, 35%) and 5-(pyridin-3-yl)carbonylimidazo[5,1-b]thiazole (31 mg, 6.7%).
7-(Pyridin-3-yl)carbonylimidazo[5,1-b]thiazole
¹H-NMR (400 MHz, CDCl₃): δ (ppm) 7.20 (1H, d), 7.45 (1H, ddd), 7.63 (1 H, d), 8.10 (1 H, s), 8.78 (1 H, dd), 8.83 (1 H, dt), 9.72 (1 H, dd); FABMS m/z 230 (M + H)⁺
5-(Pyridin-3-yl)carbonylimidazo[5,1-b]thiazole
¹H-NMR (400 MHz, CDCl₃): δ (ppm) 7.21 (1 H, d), 7.46 (1 H, dd), 7.48 (1 H, d), 8.72 (1 H, dd), 8.78 - 8.83 (2H, m), 9.63 (1 H, dd); FABMS m/z 230 (M + H)⁺

### Example 2

### 2-Bromo-7-(ovridin-3-yl)carbonylimidazo[5,1-b]thiazole (step (a))

N,N-Diethylnicotinamide (890 g, 5.0 mol) was dissolved in nitrobenzene (125 ml) under a nitrogen atmosphere, and phosphorus oxychloride (460 g, 3.0 mol) was added to the solution at room temperature. A nitrobenzene solution (750 ml) of 2-bromo-imidazo[5,1-b]thiazole (220 g, 1.0 mol) was added thereto, and the mixture was stirred at 85°C for 2 hr. The reaction solution was added to a cooled aqueous solution (16 L) of sodium acetate (250 g, 3.0 mol), and the mixture was adjusted to pH 2 by the addition of a 20% aqueous sodium acetate solution. The mixture was washed twice with ethyl acetate (7.5 L) and was adjusted to pH 10 by the addition of a 10 N aqueous sodium hydroxide solution (1.6 L) to the aqueous layer, followed by extraction with an ethyl acetate/methanol (3/1) mixed solvent. The organic layer was filtered, and the filtrate was concentrated to a volume of 1.3 L under the reduced pressure. Thereafter, water (3.65 L) was added, and the resultant precipitate was washed with water. Isopropyl alcohol/water (6.0 L/0.74 L) was added thereto, and the mixture was heated to 50°C to dissolve the precipitate. Activated carbon (65 g) and isopropyl alcohol (1.4 L) were added to the solution, and the mixture was stirred for 30 min and was filtered through Celite. The filtrate was concentrated to 2.0 L under the reduced pressure and was then ice cooled. The resultant precipitate was collected by filtration and was washed with a cold 33% aqueous isopropyl alcohol solution. The solid thus obtained was dried to give 2-bromo-7-(pyridin-3-yl)carbonylimidazo[5,1-b]thiazole (99 g, 32%).
¹H-NMR (400 MHz, CDCl₃): δ (ppm) 7.47 (1H, ddd), 7.69 (1H, s), 8.04 (1 H, s), 8.78 (1 H, dd), 8.82 (1 H, dt), 9.73 (1 H, dd); FABMS m/z 308, 310 (M + H)⁺

### Example 3

### 2-Bromo-7-(pyridin-3-yl)carbonylimidazo[5,1-b]thiazole (step (b))

2-Bromoimidazothiazole (20.0 g, 98.5 mmol) and nicotinoyl chloride hydrochloride (87.7 g, 492 mmol) were suspended in dichloroethane (200 g) under a nitrogen atmosphere. Titanium tetrachloride (93.4 g, 492 mmol) was added dropwise to the suspension at a reflux temperature (86°C) over a period of about 20 min, and, in this state, a reaction was allowed to proceed at the reflux temperature for 57 hr. Thereafter, the mixture was cooled to 40°C, and methanol (94.7 g, 295 mmol) was added dropwise thereto over a period of about 30 min. After stirring for 30 min, the mixture was cooled to 20°C, and the solid thus obtained was collected by filtration. The solid was washed with methanol to give 2-bromo-7-(pyridin-3-yl)carbonylimidazo[5,1-b]thiazole hydrochloride as a light yellow product (30.1 g, yield 72.6%, monohydrochloride).
¹H-NMR (300 MHz, DMSO-d6, TMS): δ (ppm) 7.85 - 7.90 (1H, t), 8.52 (1 H, s), 8.54 (1 H, s), 8.93 (1 H, dd), 8.97 - 9.00 (1 H, m), 9.70 (1 H, s); EIMS m/z 307.92 (M + H)⁺

### Example 4

### 2-Ethoxycarbonyl-7-(pyridin-3-yl)carbonylimidazo[5,1-b]thiazole ( step (a))

N,N-Diethylnicotinamide (1.8 g, 10.1 mmol) was dissolved in nitrobenzene (2.0 ml) under an argon atmosphere, and phosphorus oxychloride (900 ml, 9.66 mmol) was added dropwise to the solution at room temperature. A nitrobenzene solution (2.0 ml) of 2-ethoxycarbonyl-imidazo[5,1-b]thiazole (400 mg, 2.04 mmol) was added thereto, and the mixture was stirred at 80°C for 3 hr. After the completion of the reaction, the reaction solution was added to a saturated aqueous sodium hydrogencarbonate solution, and the mixture was subjected to separation with ethyl acetate. The aqueous layer was allowed to stand at room temperature for one day, and the resultant precipitate was collected by filtration. The solid was purified by column chromatography on silica gel (ethyl acetate) to give 2-ethoxycarbonyl-7-(pyridin-3-yl)carbonylimidazo[5,1-b]thiazole (185 mg, 30%).

Separately, the organic layer was dried over anhydrous magnesium sulfate, and the residue was purified by column chromatography on silica gel (hexane/ethyl acetate = 1/1) to give 2-ethoxycarbonyl-5-(pyridin-3-yl)carbonylimidazo[5,1-b]thiazole (51 mg, 8.3%).
2-Ethoxycarbonyl-7-(pyridin-3-yl)carbonylimidazo[5,1-b]thiazole
¹H-NMR (400 MHz, CDCl₃): δ (ppm) 1.43 (3H, t), 4.45 (2H, q), 7.46 (1H, ddd), 8.15 (1 H, s), 8.28 (1 H, s), 8.79 (1 H, dd), 8.82 (1 H, dt), 9.74 (1 H,
dd); EIMS m/z 301
2-Ethoxycarbonyl-5-(pyridin-3-yl)carbonylimidazo[5,1-b]thiazole
¹H-NMR (400 MHz, CDCl₃): δ (ppm) 1.44 (3H, t), 4.46 (2H, q), 7.44 - 7.51 (2H, m), 8.79 - 8.86 (2H, m), 9.29 (1 H, d), 9.66 (1 H, dd); EIMS m/z 301

### Example 5

### 2-Ethoxycarbonyl-7-(pyridin-3-yl)carbonylimidazo[5,1-b]thiazole ( step (b))

2-Ethoxycarbonyl-imidazo[5,1-b]thiazole (200 mg, 1.0 mmol) and nicotinoyl chloride (1.78 g, 10 mmol) were added to and suspended in 1,2-dichloroethane (5.0 ml) under an argon atmosphere. Titanium tetrachloride (3.3 ml, 30 mmol) was added thereto under ice cooling, and the mixture was refluxed for 8 hr. An aqueous sodium hydrogencarbonate solution was added to stop the reaction, followed by extraction with ethyl acetate. The organic layer was analyzed by high-performance liquid chromatography (column: Cosmosil 4.6 x 150 mm, development system: acetonitrile/phosphate buffer = 4/6) to give 2-ethoxycarbonyl-7-(pyridin-3-yl)carbonylimidazo[5,1-b]thiazole (reaction yield 77%).

### Example 6

### 2-Propionyl-7-(pyridin-3-yl)carbonylimidazo[5,1-b]thiazole (step ( a))

N,N-Dimethylnicotinamide (610 mg, 4.10 mmol) was dissolved in 1,2-dichloroethane (1.0 ml) under an argon atmosphere, and phosphorus oxychloride (1.27 g, 8.30 mmol) was added dropwise to the solution at room temperature. A 1,2-dichloroethane solution (3.0 ml) of 2-propionyl-imidazo[5,1-b]thiazole (360 mg, 2.00 mmol) was added thereto, and the mixture was refluxed for 16 hr. A 1 N aqueous sodium hydroxide solution was added to stop the reaction, and the reaction mixture was extracted with dichloroethane/methanol (5/1) mixed solvent. The organic layer was concentrated under the reduced pressure. The residue was purified by column chromatography on silica gel (development system: ethyl acetate/methanol = 10/1) to give 2-propionyl-7-(pyridin-3-yl)carbonylimidazo[5,1-b]thiazole (160 mg, 28%) and 2-propionyl-5-(pyridin-3-yl)carbonylimidazo[5,1-b]thiazole (30 mg, 11%). ¹H-NMR (400 MHz, CDCl₃): δ (ppm) 1.29 (3H, t), 2.96 (2H, q), 7.46 (1H, ddd), 8.16 (1 H, s), 8.21 (1, s), 8.78 - 8.83 (2H, m), 9.75 (1 H, dd); FABMS m/z 286 (M + H)⁺

### Preparation 1

### 5-Ethoxycarbonyl-2-formylaminomethylthiazole (compound of formula (c) in scheme D)

A compound (5 g, 42.37 mmol) of formula (a) in scheme D synthesized by the method described in WO 98/29139 was dissolved in DMF (50 ml). Sodium bromide (15.2 g) was introduced into this solution, and the solution temperature was brought to 40°C. In another egg-plant type flask, N-(thiocarbamoylmethyl)formamide (formula (b) in scheme D) (28 g, 148.94 mmol) was dissolved in DMF (100 ml). The solution was cooled to 0°C, and a 4 N hydrogen chloride/dioxane solution (19 ml) was added dropwise thereto over a period of 15 min. While maintaining the solution of compound of formula (a) in scheme D at 40°C, the mixed solution of N-(thiocarbamoylmethyl)formamide was added dropwise over a period of one hr, and the reaction solution was stirred for 3 hr while maintaining the temperature of the reaction solution at 40°C. After the completion of the reaction, DMF was removed by about 80 ml under the reduced pressure, and the precipitate formed in the residual solution was collected by filtration. A saturated aqueous sodium bicarbonate solution (50 ml) and 130 ml of water were introduced into the mother liquor, and extraction was repeated twice with 150 ml of ethyl acetate. The organic layer was washed twice with 15% brine and was dried over Na₂SO₄. Activated carbon (1 g) was introduced into the mother liquor, and the mixture was stirred for one hr and was then filtered. The mother liquor was concentrated to 40 ml under the reduced pressure, and the solution was cooled to 0°C for crystallization. Hexane (10 ml) was introduced, and the reaction solution was then kept at 0°C with stirring for 24 hr. The resultant crystal was collected by filtration and was dried under the reduced pressure to give 5-ethoxycarbonyl-2-formylaminomethylthiazole (a compound of formula (c) in scheme D) (3.47 g, 38%).
¹H-NMR (400 MHz, CDCl₃) : δ (ppm) 1.33 (3H, t), 4.31 (1 H, q), 4.74 (2H, d), 6.84 (1 H, brs), 8.23 (1 H, s), 8.28 (1 H, s)

### Preparation 2

### 2-Ethoxycarbonylimidazo[5,1-b]thiazole (a compound of formula (d) in scheme D)

5-Ethoxycarbonyl-2-formylaminomethylthiazole (a compound of formula (c) in scheme D) (5.9 g, 23.69 mmol) and toluene (60 ml) were introduced. The mixture was heated to 60°C. A mixed liquid composed of phosphorus oxychloride (5.44 g) and toluene (12 ml) was added dropwise thereto, and the mixture was stirred for one hr. After the completion of the reaction, the mixture was cooled to 0°C, and 50 ml of a 0.5 N aqueous hydrochloric acid solution was introduced followed by separation. The aqueous layer was washed with toluene (30 ml) and was then cooled to 0°C. The aqueous layer was adjusted to pH 6.9 by the addition of a 5 N aqueous sodium hydroxide solution for crystallization. The resultant crystal was collected by filtration, was washed with 10 ml of water, and was dried under the reduced pressure to give 2-ethoxycarbonylimidazo[5,1-b]thiazole (a compound of formula (d) in scheme D) (5.02 g, 97%).
¹H-NMR (400 MHz, CDCl₃): δ (ppm) 1.39 (3H, t), 4.40 (1H, q), 7.11 (1H, s), 8.07 (1 H, s), 8.11 (1 H, s)

### Preparation 3

### 2-Bromo-7-dimethoxy(pyridin-3-yl)methylimidazo[5,1-b]thiazole ( a compound of formula (B))

Methanol (1.5 L) and methyl orthoformate (2.39 kg, 22.5 mol) were added to and suspended in 2-bromo-7-(pyridin-3-yl)carbonylimidazo[5,1-b]thiazole (235 g, 0.75 mol). Tosylic acid monohydrate (1.44 kg, 7.5 mol) was added to the suspension, and the mixture was stirred at 60°C for 19 hr. Methyl orthoformate (400 g, 3.75 mol) was added thereto every two hr three times in total. A 28% aqueous sodium methoxide methanol solution (2.35 L, 9.0 mol) was added under ice cooling to stop the reaction. Methanol (580 ml) was added thereto, and the mixture was stirred at room temperature and was filtered through Celite. Water (1.5 L) was added to the filtrate, and the mixture was concentrated under the reduced pressure to a volume of 2.2 L. 10% brine was added thereto, and the mixture was extracted with ethyl acetate, followed by washing with 10% brine. The organic layer was dried over anhydrous magnesium sulfate and was concentrated under the reduced pressure to a volume of 750 ml. The residue was stirred at room temperature, and the resultant precipitate was collected by filtration, was washed with cold ethyl acetate, and was dried to give 2-bromo-7-dimethoxy(pyridin-3-yl)methylimidazo[5,1-b]thiazole (190 g, 70%).
¹H-NMR (400 MHz, CDCl₃): δ (ppm) 3.21 (6H, s), 7.24 - 7.29 (1H, m), 7.44 (1 H, s), 7.90 (1 H, ddd), 8.52 (1 H, dd), 8.73 (1 H, d)

### Preparation 4

### 2-Ethoxycarbonyl-7-dimethoxy(pyridin-3-yl)methylimidazo[5,1-b]thiazole (a compound of formula (C) in scheme E)

2-Ethoxycarbonyl-7-(pyridin-3-yl)carbonylimidazo[5,1-b]thiazole (1.0 g, 3.7 mmol) was dissolved in tetrahydrofuran (50 ml). A 5 N aqueous sodium hydroxide solution (30 ml) was added to the solution, and the mixture was heated to 50°C. The heated mixture was vigorously stirred for one hr, was then cooled to room temperature, followed by separation with ethyl acetate. The aqueous layer was adjusted to pH 4 by the addition of 1 N hydrochloric acid and was then cooled. The resultant precipitate was collected by filtration, was washed with water, and was dried to give 2-carboxyl-7-(pyridin-3-yl)carbonylimidazo[5,1-b]thiazole.

2-Carboxyl-7-(pyridin-3-yl)carbonylimidazo[5,1-b]thiazole (11.5 g, 42 mmol) was suspended in methanol (230 ml). p-Toluenesulfonic acid monohydrate (80.7 g, 420 mmol) and methyl orthoformate (140 ml, 1.3 mol) were added to the suspension, and the mixture was refluxed for one day. Thereafter, methyl orthoformate (40 ml) and methanol (20 ml) were added thereto, and the mixture was further refluxed. Nine hr after the initiation of the reflux, the mixture was poured into a 28% sodium methoxide/methanol solution (100 g) under ice cooling to stop the reaction. The resultant precipitate was collected by filtration, the organic layer was concentrated under the reduced pressure, and the residue was dissolved in ethyl acetate. After separation with a phosphate buffer solution, the organic layer was dried over anhydrous magnesium sulfate and was concentrated under the reduced pressure. The residue was dissolved in a methanol/tetrahydrofuran (35 ml/70 ml) mixed solvent. A 1 N aqueous sodium hydroxide solution (35 ml) was added to the solution, and the mixture was vigorously stirred for 2 hr. The organic solvent was removed by distillation under the reduced pressure, and the residue was lyophilized to give 2-carboxyl-7-dimethoxy(pyridin-3-yl)methylimidazo[5,1-b]thiazole sodium salt.

Tetrahydrofuran (63 ml) was added to and suspended in 2-carboxyl-7-dimethoxy(pyridin-3-yl)methylimidazo[5,1-b]thiazole sodium salt (2.1 g) under an argon atmosphere, and pivaloyl chloride (1.2 ml) was added dropwise to the suspension under ice cooling. After the confirmation of disappearance of 2-carboxyl-7-dimethoxy(pyridin-3-yl)methylimidazo[5,1-b]thiazole by high-performance liquid chromatography (acetonitrile/phosphate buffer solution = 6/4), the mixture was cooled to -78°C, and ethylmagnesium bromide (0.86 M, 14.6 ml) was added dropwise thereto. The mixture was stirred for 2 hr, and a saturated aqueous ammonium chloride solution was added to stop the reaction. The mixture was then extracted with ethyl acetate. The organic layer was dried over anhydrous magnesium sulfate and was concentrated under the reduced pressure. The residue was then purified by column chromatography on silica gel (methanol/ethyl acetate = 1/10), and the product was recrystallized from ethyl acetate to give 2-ethoxycarbonyl-7-dimethoxy(pyridin-3-yl)methylimidazo[5,1-b]thiazole (510 mg, 25%).

### Preparation 5

### Synthesis of 2-propionyl-imidazo[5,1-b]thiazole

A tetrahydrofuran solution (200 ml) of 2-bromo-imidazo[5,1-b]thiazole (16.5 g, 81 mmol) was cooled to -30°C under an argon atmosphere. Ethyl magnesium bromide (0.89 M, 100 ml) was added thereto, and the mixture was stirred for 40 min. A tetrahydrofuran solution (100 ml) of N-methyl-N-methoxypropionamide (10.5 g, 90 mmol) was added thereto, the temperature of the mixture was raised to 15°C, followed by stirring for 3.5 hr. A saturated aqueous ammonium chloride solution was added thereto to stop the reaction. The mixture was extracted with ethyl acetate, was dried over anhydrous magnesium sulfate, and was then concentrated under the reduced pressure. The residue was purified by column chromatography on silica gel (ethyl acetate → ethyl acetate/methanol = 10/1) to give 2-propionylimidazo[5,1-b]thiazole (11.8 g, 80%).
¹H-NMR (400 MHz, CDCl₃): δ (ppm) 1.26 (3H, t), 2.89 (2H, q), 7.11 (1H, s), 8.06 (1 H, s), 8.09 (1 H, s); FABMS m/z 181 (M + H)⁺

## Claims

1. A process for producing a compound of formula (I) 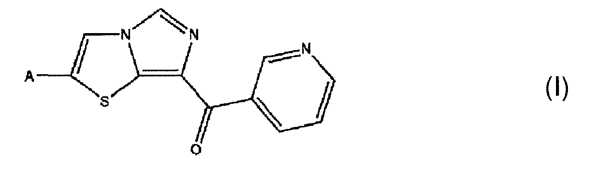 wherein A represents a hydrogen atom, a halogen atom, or -COR¹ wherein R¹ represents C₁₋₁₂ alkyl or C₁₋₁₂ alkyloxy, the process comprising reacting a compound of formula (II): 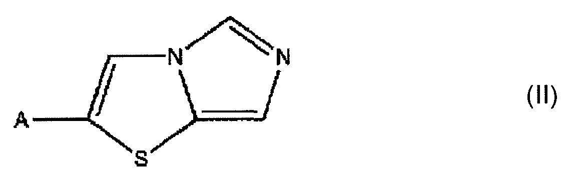 wherein A is as defined above, with a compound of formula (III): 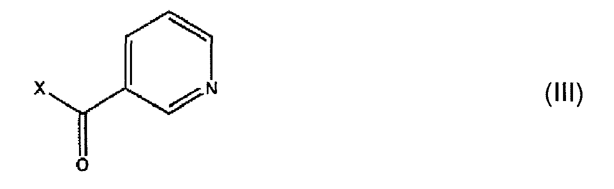 wherein X represents -NR²R³ in which R² and R³, which may be the same or different, represent C₁₋₁₂ alkyl, or R² and R³ together represent -(CH₂)n- wherein n is an integer of 2 to 8; or a halogen atom.

2. The process according to claim 1, wherein the compound of formula (II) and the compound of formula (III) (wherein X represents -NR²R³) are reacted with each other in the presence of a halogenating reagent.

3. The process according to claim 1, wherein the compound of formula (II) and the compound of formula (III), wherein X represents a halogen atom, are reacted with each other in the presence of a Lewis acid.

4. The process according to claim 3, wherein, after the completion of the reaction, a lower alcohol is added to the reaction solution and the resultant compound of formula (I) is taken out as a hydrohalide.

5. The process according to claim 3 or 4, wherein the Lewis acid is aluminum chloride, titanium tetrachloride, or tin chloride.

6. The process according to any one of claims 3 to 5, wherein the Lewis acid is titanium tetrachloride.

7. The process according to any one of claims 4 to 6, wherein the lower alcohol is methanol or ethanol.
